# EUROPÄISCHE PATENTSCHRIFT

(11) **EP 2 911 571 B1**
(45) Veröffentlichungstag und Bekanntmachung des Hinweises auf die Patenterteilung: **20.03.2019**
(21) Anmeldenummer: 13774610.3
(22) Anmeldetag: 07.10.2013
(51) Int. Cl.: A61B 1/06, A61B 1/07, G02B 23/24, A61B 1/00

(54) **ENDOSKOP MIT SEITLICHER ILLUMINATION, VERWENDUNG UND VERFAHREN**
ENDOSCOPE WITH LATERAL ILLUMINATION, USE AND METHOD
ENDOSCOPE À ILLUMINATION LATÉRALE, UTILISATION ET PROCÉDÉ

(30) Priorität: 24.10.2012 DE 102012219434
(43) Veröffentlichungstag der Anmeldung: 02.09.2015
(73) Patentinhaber: Olympus Winter & Ibe GmbH, 22045 Hamburg (DE)
(72) Erfinder: SCHOUWINK, Peter Dr, 22926 Ahrensburg (DE)
(74) Vertreter: Seemann & Partner Patentanwälte mbB
(86) Internationale Anmeldenummer: PCT/EP2013/003004
(87) Internationale Veröffentlichungsnummer: WO 2014/063781

(56) Entgegenhaltungen:
- DE-A1-102004 023 024
- US-A1- 2007 185 386
- US-A1- 2010 053 312
- US-A1- 2012 184 811

## Beschreibung

Die Erfindung betrifft ein Endoskop mit seitlicher Illumination, mit einem längserstreckten Endoskopschaft mit einem Außenrohr, in dem distal eine Eintrittsoptik mit in wenigstens einer Schwenkebene verschwenkbarer Blickrichtung angeordnet ist, wobei im Endoskopschaft ein oder mehrere Illuminationslichtleitfaserbündel angeordnet sind, die an einer distalen Spitze des Endoskopschafts an Austrittsflächen enden, wobei wenigstens ein erstes Illuminationslichtleitfaserbündel eine Illumination eines vor der distalen Spitze des Endoskopschafts liegenden Bereichs unter einem 0°-Blickwinkel erzeugt und wenigstens ein zweites Illuminationslichtleitfaserbündel eine Illumination eines anderen Bereichs mit einem seitlichen Blickwinkel oder seitlichen Blickwinkelbereich zwischen 0° und 90° erzeugt. Die Erfindung betrifft ferner eine Verwendung sowie ein Verfahren zum Betreiben eines Endoskops.

Es handelt sich erfindungsgemäß um optische Endoskope, bei denen ein Seitwärtsblick eingestellt oder einstellbar ist. Derartige Endoskope weisen entweder ein optisches System mit einem Bildsensor auf, der im Endoskopschaft oder an der distalen Spitze des Endoskopschafts angeordnet ist, oder Stablinsen-Umkehrsätze, die das an der distalen Spitze eingefangene Licht zum proximalen Ende des Endoskops zu einem Okular und ggf. zu einem Videokopf weiterleiten.

Ein Blickrichtungswechsel erfolgt üblicherweise durch die Verschwenkung eines Prismas hinter einem gekrümmten Abdeckglas, welches das distale Ende des Endoskopschafts abdichtet.

Wenn eine Beleuchtung durch den Endoskopschaft erforderlich ist, weist der Endoskopschaft neben dem optischen System auch noch Mittel zur Beleuchtung, also zur Illumination des Bereiches vor der distalen Spitze des Endoskopschafts auf. Hierbei handelt es sich in einigen Fällen um Bündel von Lichtleitfasern, die proximal Licht von einer Lichtquelle, beispielsweise von Leuchtdioden (LEDs) oder anderen Lichtquellen, aufnehmen und dieses Licht nach distal zur Endoskopspitze leiten und dort zur Illumination des Operationsfeldes ausstrahlen.

Lichtleitfasern in Endoskopoptiken haben einen sehr großen Abstrahlwinkel, sollen aber mit ihrer Strahlrichtung nach Möglichkeit alle auf die Mitte des Blickfeldes gerichtet sein. In Strahlrichtung abweichende Fasern bewirken störende Helligkeitsunterschiede im Blickfeld. Zur Abdichtung und zur Sicherung ihrer während der Montage gegebenen Ausrichtung sind die Fasern im distalen Endbereich üblicherweise verklebt.

Eine entsprechende Endoskopoptik mit oberen und seitlichen Lichtleitfaserteilbündeln ist aus DE 10 2004 023 024 A1 bekannt. Hierbei handelt es sich um eine Optik mit einem Seitwärtsblick, wobei ein zentrales, asymmetrisch im Endoskopschaft angeordnetes Fenster zur Aufnahme der empfangenden Optik vorgesehen ist, und die asymmetrischen Randgebiete durch mehrere Lichtleitfaserbündel besetzt sind, von denen zwei seitliche Lichtleitfaserbündel eine seitliche Illumination unter einem Blickwinkel von ca. 45° und ein zentrales oberes Lichtleitfaserbündel eine Illumination unter einem 0°-Winkel erzeugt.

Eine zufriedenstellende Lösung für Endoskope mit verschwenkbarer Optik, die auch Seitwärtsblickrichtungen haben, bis beispielsweise 90°, ist jedoch noch nicht gefunden. Es ist daher die Aufgabe der vorliegenden Erfindung, eine ausreichende Ausleuchtung eines Operationsfeldes auch bei Endoskopen mit einstellbarer Blickrichtung zu verwirklichen.

Diese Aufgabe wird gelöst durch ein Endoskop mit seitlicher Illumination, mit einem längserstreckten Endoskopschaft mit einem Außenrohr, in dem distal eine Eintrittsoptik mit in wenigstens einer Schwenkebene verschwenkbarer Blickrichtung angeordnet ist, wobei im Endoskopschaft ein oder mehrere Illuminationslichtleitfaserbündel angeordnet sind, die an einer distalen Spitze des Endoskopschafts an Austrittsflächen enden, wobei wenigstens ein erstes Illuminationslichtleitfaserbündel eine Illumination eines vor der distalen Spitze des Endoskopschafts liegenden Bereichs unter einem 0°-Blickwinkel erzeugt und wenigstens ein zweites Illuminationslichtleitfaserbündel eine Illumination eines anderen Bereichs mit einem seitlichen Blickwinkel oder seitlichen Blickwinkelbereich zwischen 0° und 90° erzeugt, das dadurch weitergebildet ist, dass eine oder mehrere Leuchtdioden im oder am Umfang des Endoskopschafts im Bereich der distalen Spitze angeordnet sind, wobei die Leuchtdiode oder Leuchtdioden in der Schwenkebene und/oder um die Schwenkebene herum angeordnet sind, um eine Illumination in einem Blickwinkel oder Blickwinkelbereich um 90° herum zu erzeugen.

Durch den Einsatz der LEDs wird der limitierende Faktor von Illuminationslichtleitfaserbündeln umgangen, dass sie nur begrenzt biegbar sind. Innerhalb des begrenzten zur Verfügung stehenden Volumens innerhalb eines Endoskopschafts ist es kaum möglich, Illuminationslichtleitfaserbündel um einen Winkel von 90° zu biegen, ohne wenigstens einzelne Lichtleitfasern des Bündels zu brechen. Die Anordnung von LEDs am Umfang des Endoskopschafts im Bereich der distalen Spitze sorgt hingegen für eine kostengünstige, robuste und ausreichend helle Illumination des seitlichen Bereiches, der durch die vorhandenen Illuminationslichtleitfaserbündel nicht oder nicht ausreichend ausgeleuchtet wird.

Vorzugsweise überlappen die vom zweiten Illuminationslichtleitfaserbündel und von der Leuchtdiode oder den Leuchtdioden illuminierten Bereiche. Auf diese Weise wird eine lückenlose Ausleuchtung des Operationsfeldes erreicht.

Zur Anordnung der Leuchtdiode oder der Leuchtdioden ist es vorteilhaft, dass die Leuchtdiode oder Leuchtdioden in das Außenrohr oder in ein Gehäuse eingebettet ist oder sind, das distal am Endoskopschaft angeordnet ist. Bei einer Einbettung in den Umfang entweder im Außenrohr oder in dem entsprechenden Gehäuse an der distalen Spitze ragt die LED oder ragen die LEDs nicht über den Umfang des Endoskopschafts hervor und bilden somit kein Hindernis. Sie können beispielsweise durch eine durchsichtige Lackierung oder durch eine durchsichtige Folie oder ein durchsichtiges Rohr geschützt sein.

Vorteilhafterweise sind mehrere Leuchtdioden symmetrisch um die Schwenkebene herum angeordnet. Die äußeren Leuchtdioden sind dabei vorteilhafterweise unabhängig von einer zentralen Leuchtdiode oder mehreren zentralen Leuchtdioden schaltbar. Auf diese Weise ist es auswählbar, wie groß der Bereich in einer seitlichen Richtung sein soll, der ausgeleuchtet werden soll. So ist es beispielsweise möglich, bei einem relativ kleinen Schwenkwinkel bzw. Blickwinkel, der in den durch die LEDs ausgeleuchteten Bereich lediglich peripher eintritt, nur die zentrale oder zentralen Leuchtdiode bzw. Leuchtdioden anzuschalten und bei Eintritt in den durch die Leuchtdioden beleuchteten Bereich auch die äußeren Leuchtdioden anzuschalten, um den Bereich vollständig auszuleuchten. So kann die Wärmeerzeugung an der Endoskopspitze gering gehalten werden.

Die Leuchtdiode oder Leuchtdioden ist oder sind vorzugsweise als flächige Leuchtdiode oder Leuchtdioden ausgebildet oder es sind mehrere Leuchtdioden in einem eine Fläche aufspannenden Muster angeordnet. Ziel ist eine gleichmäßige Ausleuchtung des Operationsbereiches. Flächige Leuchtdioden bilden hierfür eine sehr homogene Quelle, die sich bei kleinen Leuchtdioden durch eine entsprechende Verteilung der einzelnen Leuchtdioden erreichen lässt. Besonders geeignet sind organische Leuchtdioden (OLED), die als flächige Leuchtdioden herstellbar sind.

Untersuchungen, beispielsweise verschiedener Gewebetypen oder Krankheitsbilder, können vorteilhafterweise ausgeführt werden, wenn eine oder mehrere Leuchtdioden zur Erzeugung von Licht mit unterschiedlichen Farben oder mit einstellbaren Farben ausgebildet sind. Dabei können die Farben solche im sichtbaren Bereich des optischen Spektrums sein oder auch Infrarot oder Ultraviolett, wobei in diesem Fall entsprechend empfindliche Bildsensoren eingesetzt werden oder diese in Verbindung beispielsweise mit Lumineszenzmarkern im Gewebe des Patienten eingesetzt werden.

Vorzugsweise ist eine Steuervorrichtung umfasst, mittels der die Illumination durch die Illuminationslichtleitfaserbündel und die Leuchtdiode oder Leuchtdioden in Abhängigkeit vom eingestellten Blickwinkel steuerbar ist, um den in der Richtung des eingestellten Blickwinkels liegenden Bereich vor der distalen Spitze des Endoskopschafts zu illuminieren. Auf diese Weise ist die Auswahl der Illuminationsquellen, also erstes Illuminationslichtleitfaserbündel, zweites Illuminationslichtleitfaserbündel und Leuchtdiode oder Leuchtdioden, nicht mehr Aufgabe des Operateurs, sondern wird automatisch anhand des eingestellten Blickwinkels gewählt.

Die der Erfindung zugrunde liegende Aufgabe wird auch durch eine Verwendung einer Leuchtdiode oder mehrerer Leuchtdioden in einem erfindungsgemäßen, zuvor beschriebenen Endoskop zur Erzeugung einer seitlichen Illumination gelöst. Hierdurch wird es möglich, auf einfache, kostengünstige und effiziente Weise eine seitliche Beleuchtung in Verschwenkungsrichtung der Optik zu erzeugen. Da nur ein seitlicher Einsatz von LEDs notwendig ist und Licht weiterhin in Geradeausrichtung und weniger stark seitlicher Richtung durch Illuminationslichtleitfaserbündel geschieht, hält sich die Wärmeerzeugung durch die lichtemittierenden Dioden in engen Grenzen und führt nicht zu einer Schädigung von Gewebe oder Organen einer zu untersuchenden oder zu operierenden Person.

Schließlich wird die der Erfindung zugrunde liegende Aufgabe auch durch ein Verfahren zum Betreiben eines erfindungsgemäßen, zuvor beschriebenen Endoskops gelöst, wobei bei einer Verschwenkung eines Blickwinkels in Abhängigkeit vom eingestellten Blickwinkel eine Illumination über wenigstens ein erstes Illuminationslichtleitfaserbündel mit 0°-Blickrichtung, über wenigstens ein zweites Illuminationslichtleitfaserbündel mit einem seitlichen Blickwinkel oder einem seitlichen Blickwinkelbereich zwischen 0° und 90° und/oder über eine oder mehrere Leuchtdioden erzeugt wird, die im oder am Umfang des Endoskopschafts im Bereich der distalen Spitze angeordnet sind, wobei die Leuchtdiode oder Leuchtdioden in der Schwenkebene und/oder um die Schwenkebene herum angeordnet sind, um eine Illumination in einem Blickwinkel oder Blickwinkelbereich um 90° herum zu erzeugen.

Die zu den einzelnen Erfindungsgegenständen, also dem Endoskop, der Verwendung und dem Verfahren genannten Vorteile, Eigenschaften und Merkmale gelten jeweils auch für die anderen Erfindungsgegenstände, die sich aufeinander beziehen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Querschnittsdarstellung durch einen Endoskopschaft eines erfindungsgemäßen Endoskops und
- Fig. 2: eine schematische Seitenansicht des Bereichs der distalen Spitze eines Endoskopschafts eines erfindungsgemäßen Endoskops.

In den Zeichnungen sind jeweils gleiche oder gleichartige Elemente und/oder Teile mit denselben Bezugsziffern versehen, so dass von einer erneuten Vorstellung jeweils abgesehen wird.

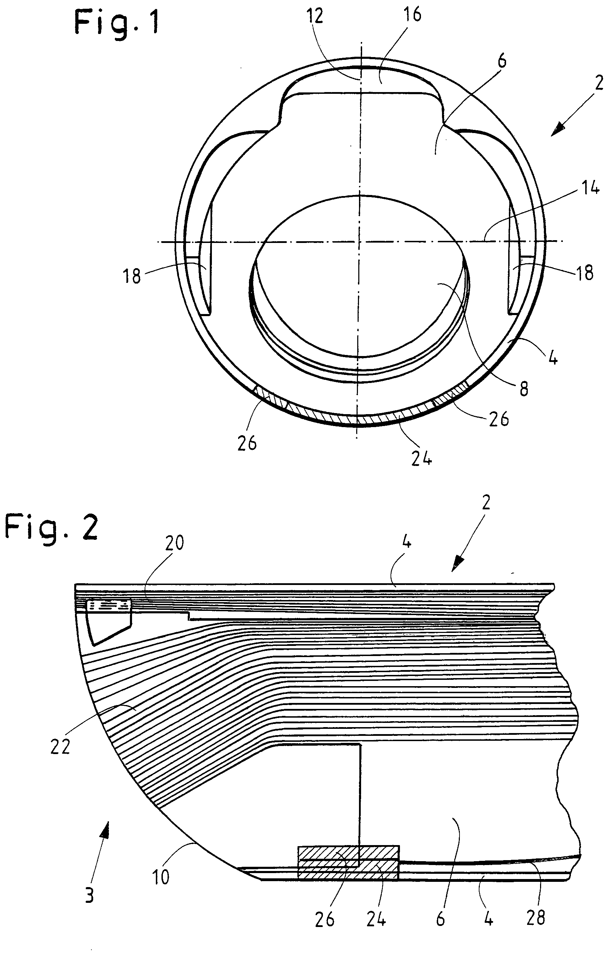

In Fig. 1 ist ein Endoskopschaft 2 eines erfindungsgemäßen Endoskops im Bereich der distalen Spitze 3 im Querschnitt schematisch dargestellt. Innerhalb eines Außenrohres 4 ist ein Faserrohr 6 angeordnet, das einerseits eine Fensteröffnung 8 für eine optische Baugruppe und andererseits eine obere Austrittsöffnung 16 und seitliche Austrittsöffnungen 18 für Illuminationslichtleitfaserbündel aufweist. Der Durchmesser der optischen Baugruppe, die in die Fensteröffnung 8 eingelassen ist, ist kleiner als der Durchmesser des Endoskopschafts 2 und ist zum Rand hin versetzt eingelassen, so dass um die Fensteröffnung 8 herum Platz für die Austrittsöffnungen 16, 18 zur Illumination eines Operationsfeldes vor der distalen Spitze des Endoskops vorhanden ist.

Die optische Baugruppe, die in die Fensteröffnung 8 einzusetzen ist, kann eine starr seitlich blickende Baugruppe sein oder eine Baugruppe mit verschwenkbarer Blickrichtung, beispielsweise mittels einer Schwenkprismenanordnung, wobei die Schwenkebene, also die Ebene, in der die Verschwenkung der Blickrichtung bzw. des Prismas stattfindet, mit dem Bezugszeichen 12 gekennzeichnet ist. Senkrecht dazu, ebenfalls quer zur Längserstreckung des Endoskopschafts 2, ist eine horizontale Achse 14 dargestellt, die auch parallel zu einer Drehachse einer entsprechenden Schwenkprismenanordnung verläuft.

Unterhalb der Fensteröffnung 8 ist im Bereich des Außenrohrs 4 eine Anzahl von Leuchtdioden 24, 26 dargestellt, die so ausgerichtet sind, dass sie einen unteren Bereich beleuchten, der teilweise vor der distalen Spitze des Endoskopschafts 2 angeordnet ist. Dabei handelt es sich um eine breite Leuchtdiode 24, die symmetrisch um die Schwenkebene 12 herum angeordnet ist, sowie zwei seitliche Leuchtdioden 26, die symmetrisch um die Schwenkebene 12 herum von der ersten Leuchtdiode 24 angeordnet sind. Hierbei kann es sich um flächige Leuchtdioden oder um Leuchtdiodenfelder mit einzelnen Leuchtdioden handeln, die die entsprechenden Flächen aufspannen.

In Fig. 2 ist der Bereich der distalen Spitze 3 des Endoskopschafts 2 gemäß Fig. 1 seitlich dargestellt, wobei in diesem Fall die Illuminationslichtleitfaserbündel 20, 22 zusätzlich dargestellt sind, die in der Ebene der Sicht hintereinander angeordnet sind. Dabei ist im oberen Teil, entsprechend der oberen Austrittsöffnung 16 aus Fig. 1, ein erstes Illuminationslichtleitfaserbündel 20 angeordnet, dass in eine 0°-Blickrichtung ausgerichtet ist und abstrahlt. Das seitliche Illuminationslichtleitfaserbündel 22 ist im Bereich der distalen Spitze 3 unter einem Winkel, im vorliegenden Fall ca. 30°, abweichend von der 0°-Blickrichtung, angeordnet und illuminiert einen in Schwenkrichtung mehr seitlich gelegenen Bereich. An der distalen Spitze 3 ist der Endoskopschaft 2 durch ein gekrümmtes Abdeckglas 10 abgeschlossen.

Im unteren Bereich des Endoskopschafts 2 sind die Leuchtdioden 24 und seitlichen Leuchtdioden 26 aus Fig. 1 von der Seite her dargestellt. Diese weisen eine vergleichsweise breite Illuminationscharakteristik auf und dienen dazu, den Bereich vor und unter der Endoskopspitze 3 zu beleuchten, der durch das Licht aus den Lichtleitfasern der zweiten Illuminationslichtleitfaserbündel 22 nicht erreicht werden. Ferner ist eine Stromleitung 28 dargestellt, die die Leuchtdioden 24, 26 von proximal mit Strom versorgt.

Mittels der weiteren Beleuchtung durch die Leuchtdioden 24, 26 ist es nunmehr möglich, einen sehr seitlichen Blickwinkel von bis zu 90° einzustellen und dabei ein voll beleuchtetes Operationsfeld zu haben. Die Anordnung der Leuchtdioden 24, 26 ist dabei so gewählt, dass der 90°-Blickwinkel der Optik frei bleibt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 2: Endoskopschaft
- 3: distale Spitze
- 4: Außenrohr
- 6: Faserrohr
- 8: Fensteröffnung
- 10: Abdeckglas
- 12: Schwenkebene
- 14: Horizontalachse
- 16: obere Austrittsöffnung
- 18: seitliche Austrittsöffnung
- 20: erstes Illuminationslichtleitfaserbündel
- 22: zweites Illuminationslichtleitfaserbündel
- 24: Leuchtdiode
- 26: seitliche Leuchtdiode
- 28: Stromleitung

## Patentansprüche

1. Endoskop mit seitlicher Illumination, mit einem längserstreckten Endoskopschaft (2) mit einem Außenrohr (4), in dem distal eine Eintrittsoptik mit in wenigstens einer Schwenkebene (12) verschwenkbarer Blickrichtung angeordnet ist, wobei im Endoskopschaft (2) mehrere Illuminationslichtleitfaserbündel (20, 22) angeordnet sind, die an einer distalen Spitze (3) des Endoskopschafts (2) an Austrittsflächen enden, wobei wenigstens ein erstes Illuminationslichtleitfaserbündel (20) eine Illumination eines vor der distalen Spitze (3) des Endoskopschafts (2) liegenden Bereichs unter einem 0°-Blickwinkel erzeugt und wenigstens ein zweites Illuminationslichtleitfaserbündel (22) eine Illumination eines anderen Bereichs mit einem seitlichen Blickwinkel oder seitlichen Blickwinkelbereich zwischen 0° und 90° erzeugt, **dadurch gekennzeichnet, dass** eine oder mehrere Leuchtdioden (24, 26) im oder am Umfang des Endoskopschafts (2) im Bereich der distalen Spitze (3) angeordnet sind, wobei die Leuchtdiode (24) oder Leuchtdioden (24, 26) in der Schwenkebene (12) und/oder um die Schwenkebene (12) herum angeordnet sind, um eine Illumination in einem Blickwinkel oder Blickwinkelbereich um 90° herum zu erzeugen.

2. Endoskop nach Anspruch 1, **dadurch gekennzeichnet, dass** die vom zweiten Illuminationslichtleitfaserbündel (22) und von der Leuchtdiode (24) oder den Leuchtdioden (24, 26) illuminierten Bereiche überlappen.

3. Endoskop nach Anspruch 1 oder 2, **dadurch gekennzeichnet, dass** die Leuchtdiode (24) oder Leuchtdioden (24, 26) in das Außenrohr (4) oder in ein Gehäuse eingebettet ist oder sind, das distal am Endoskopschaft (2) angeordnet ist.

4. Endoskop nach einem der Ansprüche 1 bis 3, **dadurch gekennzeichnet, dass** mehrere Leuchtdioden (24, 26) symmetrisch um die Schwenkebene (12) herum angeordnet sind.

5. Endoskop nach Anspruch 4, **dadurch gekennzeichnet, dass** die äußeren Leuchtdioden unabhängig von einer zentralen Leuchtdiode oder mehreren zentralen Leuchtdioden schaltbar sind.

6. Endoskop nach einem der Ansprüche 1 bis 5, **dadurch gekennzeichnet, dass** die Leuchtdiode (24) oder Leuchtdioden (24, 26) als flächige Leuchtdiode oder Leuchtdioden ausgebildet ist oder sind oder mehrere Leuchtdioden in einem eine Fläche aufspannenden Muster angeordnet sind.

7. Endoskop nach einem der Ansprüche 1 bis 6, **dadurch gekennzeichnet, dass** eine oder mehrere Leuchtdioden (24, 26) zur Erzeugung von Licht mit unterschiedlichen Farben oder mit einstellbaren Farben ausgebildet sind.

8. Endoskop nach einem der Ansprüche 1 bis 7, **dadurch gekennzeichnet, dass** eine Steuervorrichtung umfasst ist, mittels der die Illumination durch die Illuminationslichtleitfaserbündel (20, 22) und die Leuchtdiode (24) oder Leuchtdioden (26) in Abhängigkeit vom eingestellten Blickwinkel steuerbar ist, um den in der Richtung des eingestellten Blickwinkels liegenden Bereich vor der distalen Spitze (3) des Endoskopschafts (2) zu illuminieren.

## Claims

1. An endoscope with lateral illumination, having an elongated endoscope shaft (2) with an outer tube (4), in the distal area of which is arranged an input lens with a viewing direction pivotable in at least one pivot plane (12), wherein the endoscope shaft (2) accommodates several illumination optical-fiber bundles (20, 22), which terminate at output surfaces at a distal tip (3) of the endoscope shaft (2), wherein at least a first illumination optical-fiber bundle (20) generates an illumination, at a 0° viewing angle, of an area lying in front of the distal tip (3) of the endoscope shaft (2), and at least a second illumination optical-fiber bundle (22) generates an illumination of another area with a lateral viewing angle or lateral viewing-angle range of between 0° and 90°, **characterized in that** one or more light-emitting diodes (24, 26) are arranged in or on the circumference of the endoscope shaft (2) in the area of the distal tip (3), wherein the light-emitting diode (24) or light-emitting diodes (24, 26) are arranged in the pivot plane (12) and/or around the pivot plane (12), in order to generate an illumination at a viewing angle or a viewing-angle range about 90°.

2. The endoscope according to claim 1, **characterized in that** the areas illuminated by the second illumination optical-fiber bundle (22) and by the light-emitting diode (24) or the light-emitting diodes (24, 26) overlap.

3. The endoscope according to claim 1 or 2, **characterized in that** the light-emitting diode (24) or light-emitting diodes (24, 26) is/are embedded in the outer tube (4) or in a housing, which is arranged distally on the endoscope shaft (2).

4. The endoscope according to one of claims 1 to 3, **characterized in that** several light-emitting diodes (24, 26) are arranged symmetrically around the pivot plane (12).

5. The endoscope according to claim 4, **characterized in that** the outer light-emitting diodes are switchable independently of a central light-emitting diode or several central light-emitting diodes.

6. The endoscope according to one of claims 1 to 5, **characterized in that** the light-emitting diode (24) or light-emitting diodes (24, 26) is or are designed as a planar light-emitting diode or light-emitting diodes or several light-emitting diodes are arranged in a pattern spanning a surface.

7. The endoscope according to one of claims 1 to 6, **characterized in that** one or more light-emitting diodes (24, 26) are designed to generate light with different colors or with adjustable colors.

8. The endoscope according to one of claims 1 to 7, **characterized in that** a control apparatus is included, by means of which the illumination by the illumination optical-fiber bundles (20, 22) and the light-emitting diode (24) or light-emitting diodes (26) is controllable depending on the set viewing angle, in order to illuminate the area lying in the direction of the set viewing angle in front of the distal tip (3) of the endoscope shaft (2).

## Revendications

1. Endoscope à éclairage latéral, comprenant une tige d'endoscope (2) s'étendant longitudinalement avec un tube extérieur (4), dans lequel est disposé, de façon distale, un système optique d'entrée ayant une direction de vue pivotante dans au moins un plan de pivotement (12), plusieurs faisceaux de fibres optiques (20, 22) d'éclairage étant disposés dans la tige d'endoscope (2) et terminés en des surfaces de sortie au niveau d'une extrémité distale (3) de la tige d'endoscope (2), au moins un premier faisceau (20) de fibres optiques d'éclairage produisant un éclairage d'une zone située devant l'extrémité distale (3) de la tige de l'endoscope (2) à un angle de vue de 0° et au moins un deuxième faisceau (22) de fibres optiques d'éclairage produisant un éclairage d'une autre zone avec un angle de vue latérale ou avec une plage angulaire latérale comprise entre 0° et 90°, **caractérisé en ce qu'**une ou plusieurs diodes électroluminescentes (24, 26) sont disposées dans ou sur la périphérie de la tige de l'endoscope (2) au niveau de la zone de l'extrémité distale (3), la diode électroluminescente (24) ou les diodes électroluminescentes (24, 26) étant disposées dans le plan de pivotement (12) et/ou autour du plan de pivotement (12) afin de produire un éclairage sur un angle de vue ou sur une plage d'angle de vue d'environ 90°.

2. Endoscope selon la revendication 1, **caractérisé en ce que** les zones éclairées par le deuxième faisceau (22) de fibres optiques d'éclairage et par la diode électroluminescente (24) ou les diodes électroluminescentes (24, 26) se chevauchent.

3. Endoscope selon la revendication 1 ou la revendication 2, **caractérisé en ce que** la diode électroluminescente (24) ou les diodes électroluminescentes (24, 26) sont enchâssées dans le tube extérieur (4) ou dans un boîtier qui est disposé de façon distale sur la tige d'endoscope (2).

4. Endoscope selon l'une des revendications 1 à 3, **caractérisé en ce que** plusieurs diodes électroluminescentes (24, 26) sont disposées symétriquement autour du plan de pivotement (12).

5. Endoscope selon la revendication 4, **caractérisé en ce que** les diodes électroluminescentes extérieures peuvent être commutées indépendamment d'une diode luminescente centrale ou de plusieurs diodes luminescentes centrales.

6. Endoscope selon l'une des revendications 1 à 5, **caractérisé en ce que** la diode électroluminescente (24) ou les diodes électroluminescentes (24, 26) est ou sont conçues sous la forme d'une diode électroluminescente plane ou de diodes électroluminescentes planes, ou plusieurs diodes électroluminescentes sont disposées selon un motif couvrant une surface.

7. Endoscope selon l'une des revendications 1 à 6, **caractérisé en ce qu'**une ou plusieurs diodes électroluminescentes (24, 26) sont conçues pour générer de la lumière de couleurs différentes ou avec des couleurs réglables.

8. Endoscope selon l'une des revendications 1 à 7, **caractérisé en ce qu'**il comprend un dispositif de commande au moyen duquel l'éclairage par les faisceaux de fibres optiques (20, 22) d'éclairage et par la diode électroluminescente (24) ou les diodes électroluminescentes (26) est apte à être commandé en fonction de l'angle de vue réglé, afin d'éclairer la zone située devant l'extrémité distale (3) de la tige d'endoscope (2) dans la direction de l'angle de vue réglé.
